# EUROPEAN PATENT APPLICATION

(11) **EP 3 144 831 A1**
(43) Date of publication of application: **22.03.2017**
(21) Application number: 16160797.3
(22) Date of filing: 17.03.2016
(51) Int. Cl.: G06F 19/00, G06F 17/30, G06F 21/62

(54) **INFORMATION PROCESSING APPARATUS, INFORMATION PROCESSING METHOD, AND PROGRAM**

(30) Priority: 15.09.2015 JP 2015181334
(71) Applicant: Fuji Xerox Co., Ltd., Minato-ku, Tokyo (JP)
(72) Inventor: HOSODA, Kento, Yokohama-shi, Kanagawa (JP)
(74) Representative: Ward, James Norman

(57) **Abstract**

An information processing apparatus includes a memory that stores document data; a creating unit that creates access control information for controlling an access to the document data; a processor that applies an electronic signature to the document data, but does not apply the electronic signature to the access control information; and a controller that controls the access to the document data by using the access control information.

## Description

### Background

### (i) Technical Field

The present invention relates to an information processing apparatus, an information processing method, and a program.

### (ii) Related Art

To prevent forging and tampering of document data, an electronic signature and time stamp processing may be occasionally applied to the document data. Also, by using access control information for controlling an access to document data, the access to the document data may be occasionally controlled.

JP-A-2014-17746 discloses a system that applies an electronic signature to an electronic document.

Medical Examination Result Report Standard, Ver. 1.0, 2015 (HL7 Japan association, Japan Association of Healthcare Information Systems Industry) discloses a clinical document standard (HL7 Clinical Document Architecture (CDA) R2) provided by Health Level Seven International (HL7) (standards for interchanging healthcare information). In the CDA standard, information provided by HL7 is recorded in XML data called CDA body. For example, testee information, report creation institution information, inspection information, interview information, etc., are recorded in the XML data. Also, medical information, such as an electronic chart and a letter of referral, is treated as an attached document of the XML data. For example, medical information is created as portable document format (PDF) data, and the PDF data is treated as the attached document of the XML data.

### Summary

In a case where an electronic signature is applied to document data and access control information, if in a case where the access control to the document data is changed, it is required to change the access control information, and to apply the electronic signature again thereto. In this case, since it is required to execute calculation of hash information and making an access to a certificate authority, processing efficiency is decreased accordingly.

An object of the invention is that in a case where the access control to the document data in which the electronic signature is applied is changed, it is not required to apply the electronic signature due to the change.

According to a first aspect of the invention, there is provided an information processing apparatus comprising: a memory that stores document data; a creating unit that creates access control information for controlling an access to the document data; a processor that applies an electronic signature to the document data, but does not apply the electronic signature to the access control information; and a controller that controls the access to the document data by using the access control information.

A second aspect of the invention is directed to the information processing apparatus according to the first aspect, wherein the document data is data relating to medical care.

A third aspect of the invention is directed to the information processing apparatus according to the first or second aspect, wherein the access control information is information indicative of a publication destination of the document data.

A fourth aspect of the invention is directed to the information processing apparatus according to any one of the first to third aspects, wherein the creating unit creates the access control information different depending on a group of a publication source of the document data.

A fifth aspect of the invention is directed to the information processing apparatus according to any one of the first to fourth aspects, wherein the processor further applies time stamp processing to the document data.

According to a sixth aspect of the invention, there is provided a program causing a computer including a memory that stores document data to function as a configuration, the configuration comprising: a creating unit that creates access control information for controlling an access to the document data; a processor that applies an electronic signature to the document data, but does not apply the electronic signature to the access control information; and a controller that controls the access to the document data by using the access control information.

According to a seventh aspect of the invention, there is provided an information processing method comprising: storing document data; creating access control information for controlling an access to the document data; applying an electronic signature to the document data, but not applying the electronic signature to the access control information; and controlling the access to the document data by using the access control information.

According to the first, second, third, sixth and seventh aspects of the invention, in a case where the access control to the document data in which the electronic signature is applied is changed, it is not required to apply the electronic signature due to the change.

According to the fourth aspect of the invention, on the basis of a publication source group of document data, access control to the document data is provided.

According to the fifth aspect of the invention, in a case where the access control to the document data in which the electronic signature or the time stamp processing is applied is changed, it is not required to apply the electronic signature or the time stamp processing due to the change.

### Brief Description of the Drawings

An exemplary embodiment of the present invention will be described in detail based on the following figures, wherein:
Fig. 1 is a block diagram showing a medical information management system according to an exemplary embodiment of the present invention;
Fig. 2 is a block diagram showing a medical information management apparatus according to this exemplary embodiment;
Fig. 3 is a block diagram showing a terminal apparatus;
Fig. 4 is an illustration for describing processing in the medical information management system;
Fig. 5 is an illustration for describing processing according to a comparative example;
Fig. 6 is an illustration for describing the processing according to the comparative example;
Fig. 7 is a block diagram showing a medical information management system according to a modification;
Fig. 8 is an illustration for describing processing in the medical information management system according to the modification;
Fig. 9 illustrates an example of a medical institution management table;
Fig. 10 illustrates an example of access control information;
Fig. 11 illustrates correspondence between document data and access control information; and
Fig. 12 is a sequence diagram for describing processing in the medical information management system according to the modification.

### Detailed Description

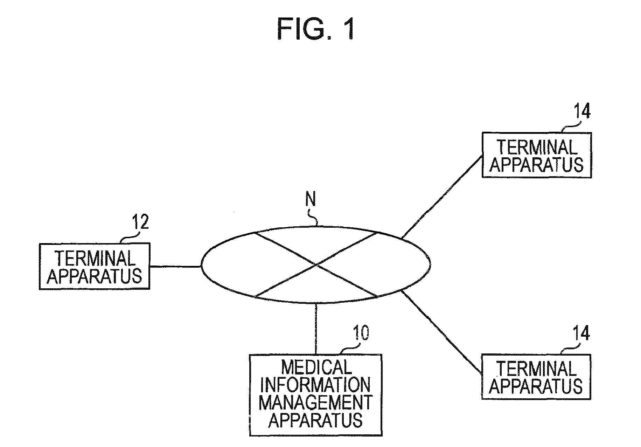

Fig. 1 illustrates an example of a medical information management system serving as an information processing system according to an exemplary embodiment of the present invention. The medical information management system includes, for example, a medical information management apparatus 10 serving as an information processing apparatus, and terminal apparatuses 12 and 14. The medical information management apparatus 10 and the terminal apparatuses 12 and 14 are connected to a communication path N such as a network.

The medical information management apparatus 10 has a function of managing document data serving as medical information and providing the document data on demand. The medical information is, for example, an electronified medical chart (an electronic chart), information indicative of a letter of referral of a medical institution, a medical image (for example, X-ray CT image, a radiographic image, etc.), information indicative of a medical fee bill, and other information. For example, data of an electronic chart, data of a letter of referral, data of a medical image, data of a medical fee bill, and other data are managed as document data. Also, if a medical chart, a letter of referral, a medical fee bill, and other documents are created in sheets of paper, images on the sheets may be read by a scanner and document data (image data) generated accordingly may be managed.

The terminal apparatuses 12 and 14 each are, for example, any of apparatuses, such as a personal computer (PC), a tablet PC, a smart phone, and a cell phone, and each have a function of transmitting and receiving data to and from other apparatuses. The terminal apparatuses 12 and 14 each are arranged in, for example, any of medical institutions, such as a university hospital, a medical office, a clinic, and a private hospital. The terminal apparatus 12 is an apparatus to be used by a publication source of document data. The terminal apparatus 14 is an apparatus to be used by a publication destination of document data. In Fig. 1, a single terminal apparatus 12 and two terminal apparatuses 14 are connected to the communication path N; however, this is merely an example. Plural terminal apparatuses 12 that are used by a publication source may be connected to the communication path N, and a single terminal apparatus 14 or plural terminal apparatuses 14 that are used by a publication destination may be connected to the communication path N.

In the medical information management system according to this exemplary embodiment, a user such as a doctor designates document data of a publication object and a publication destination of the document data by using the terminal apparatus 12. The medical information management apparatus 10 creates access control information for controlling an access to the document data in accordance with the designation. Also, if a user such as a doctor makes a request for an access to document data by using the terminal apparatus 14, the medical information management apparatus 10 controls the access to the document data by using access control information.

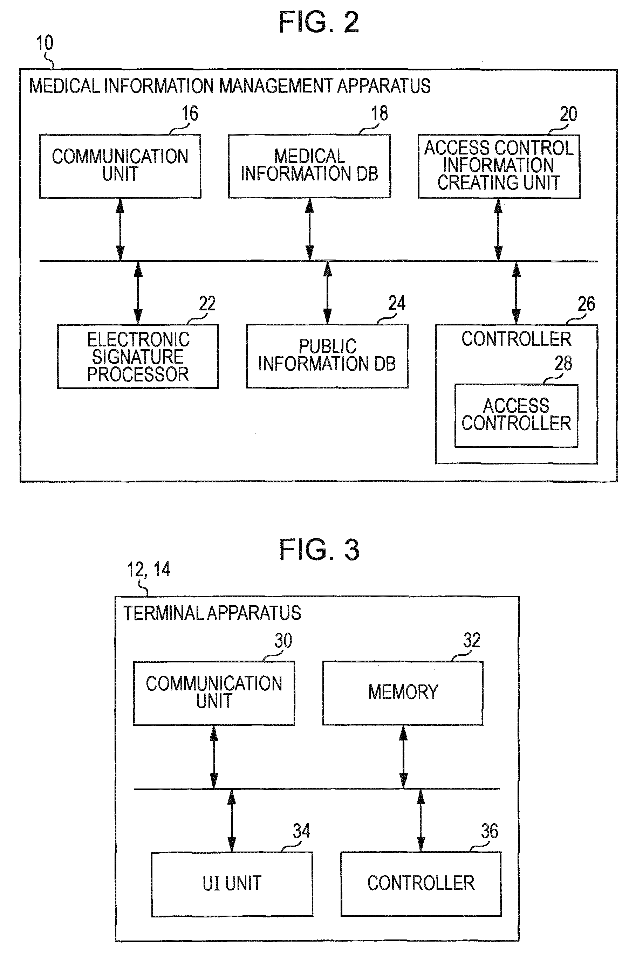

A configuration of the medical information management apparatus 10 is described below in detail with reference to Fig. 2. Fig. 2 illustrates the configuration of the medical information management apparatus 10.

A communication unit 16 is a communication interface, and has a function of transmitting data to other apparatuses and a function of receiving data from other apparatuses.

A medical information database (medical information DB) 18 is a memory device such as a hard disk. The medical information DB 18 stores document data serving as medical information. The document data is associated with document management information for managing the document data. The document management information includes, for example, document identification information for identifying the document data (for example, a document ID), information indicative of the version of the document data (for example, a version number), document type information indicative of the type of a document (for example, a document type code), information indicative of the title of the document, and information indicative of the created date of the document data. Also, the document data is associated with patient information. The patient information includes, for example, patient identification information for identifying a patient (for example, a name or a patient ID), information indicative of the sex of the patient, information indicative of the birth date of the patient, and information indicative of the age of the patient. For example, every time when document data such as an electronic chart is created, the document data is stored in the medical information DB 18. The document data stored in the medical information DB is shared by a publication source. That is, the document data stored in the medical information DB is data permitted to be accessed from the terminal apparatus 12.

For example, the medical information DB 18 stores medical information conforming to the clinical document standard (HL7 Clinical Document Architecture (CDA) R2) provided by Health Level Seven International (HL7) (standards for interchanging healthcare information). In the CDA standard, information provided by HL7 is recorded in XML data called CDA Body. For example, testee information (patient information), report creation institution information, inspection information, interview information, etc., are recorded in the XML data. Also, medical information, such as an electronic chart and a letter of referral, is treated as an attached document of the XML data. For example, medical information (document data) is created as portable document format (PDF) data, and the PDF data is treated as an attached document of the XML data. If an electronic signature is applied to the PDF data, hash information relating to the electronic signature is written in the XML data. Also, an electronic signature is applied to the XML data by XML Advanced Electronic Signatures (XAdES) etc.

Also, the medical information DB 18 stores medical institution information relating to a medical institution registered in the medical information management system. The medical institution is a medical institution of a publication destination candidate of the document data. The medical institution information includes, for example, medical institution identification information for identifying the medical institution (for example, an institution name or an institution ID). Alternatively, the medical institution information may be stored in another memory device.

The medical information DB 18 may not be provided in the medical information management apparatus 10, and may be provided in another apparatus connected to the communication path N.

An access control information creating unit 20 has a function of creating access control information (access control list, ACL) for controlling an access to document data of a publication object (for example, PDF data). The access control information creating unit 20 creates access control information, for example, every document data. The access control information includes publication destination information relating to a publication destination. The publication source and the publication destination each may be, for example, a medical institution, a department in the medical institution, or an area of a prefecture or a municipality.

The publication destination information includes, for example, publication destination identification information for identifying a publication destination (for example, a publication destination name or a publication destination ID). If the unit of the publication destination is a medical institution, such as a hospital, a medical office, or a clinic, the publication destination information includes, as the publication destination identification information, medical institution identification information for identifying the medical institution (for example, a medical institution name or a medical institution ID). If the unit of the publication destination is a department, the publication destination information includes, as the publication destination identification information, medical institution identification information for identifying a medical institution to which the department belongs to (for example, a medical institution name or a medical institution ID) and department identification information for identifying the department (for example, a department name or a department ID). If the unit of the publication destination is an area, the publication destination information includes, as the publication destination identification information, area identification information for identifying the area (for example, an area name or an area ID). The publication destination information is previously transmitted from the publication destination to the medical information management apparatus 10, and is previously stored in the medical information DB 18 of the medical information management apparatus 10. For example, when a medical institution or a department of a publication destination candidate is registered in the medical information management system, publication destination information is transmitted from the publication destination candidate to the medical information management apparatus 10 and is stored.

For example, if a user such as a doctor designates document data of a publication object and a publication destination of the document data by using the terminal apparatus 12 in a publication source, the access control information creating unit 20 creates access control information for controlling an access to the document data in accordance with the designation.

The access control information creating unit 20 associates the document data of the publication object with the access control information for the document data, and stores the document data and the access control information in the public information database (public information DB) 24. In the case conforming to the CDA standard, XML data, PDF data serving as attached document data, and access control information (ACL) are associated with each other and stored in the public information DB 24.

An electronic signature processor 22 has a function of applying an electronic signature to document data. As the electronic signature, for example, a known technique is applied. In the case conforming to the CDA standard, the electronic signature processor 22 applies an electronic signature to PDF data serving as document data, and applies the electronic signature to XML data. When the electronic signature is applied to the PDF data, hash information relating to the electronic signature is written in the XML data. Alternatively, the electronic signature is applied to the XML data by XAdES etc. The electronic signature processor 22 does not apply the electronic signature to access control information (ACL).

Also, the electronic signature processor 22 may apply time stamp processing to document data. For example, known time stamp processing is applied. In the case conforming to the CDA standard, the electronic signature processor 22 applies time stamp processing to PDF data serving as document data, and applies the time stamp processing to XML data. The electronic signature processor 22 does not apply the time stamp processing to access control information (ACL).

The public information DB 24 is a memory device such as a hard disk. The public information DB 24 stores document data of a publication object and access control information (ACL) for the document data in an associated manner. An electronic signature and time stamp processing are applied to the document data. In the case conforming to the CDA standard, XML data, PDF data serving as attached document data, and access control information (ACL) are associated with each other and stored in the public information DB 24. An electronic signature and time stamp processing are applied to the XML data and the PDF data. The electronic signature or the time stamp processing is not applied to the access control information (ACL).

A controller 26 has a function of controlling operations of respective units of the medical information management apparatus 10. Also, the controller 26 includes an access controller 28.

The access controller 28 has a function of controlling an access to document data (for example, PDF data) by using access control information (ACL). For example, if a user such as a doctor makes a request for an access to document data by using the terminal apparatus 14, the access controller 28 controls the access to the document data by using access control information associated with the document data being an object of the access request. If the access request is an access request from a publication destination, the access to the document data is permitted. In contrast, if the access request is an access request not from a publication destination, the access to the document data is inhibited.

A configuration of each of the terminal apparatuses 12 and 14 is described below in detail with reference to Fig. 3. Fig. 3 illustrates the configuration of the terminal apparatus 12. The terminal apparatus 14 has the same configuration as the terminal apparatus 12. A communication unit 30 is a communication interface, and has a function of transmitting data to other apparatuses and a function of receiving data from other apparatuses. A memory 32 is a memory device such as a hard disk. A UI unit 34 is a user interface, and includes a display and an operation unit. The display is a display device such as a liquid crystal display. The operation unit is an input device, such as a touch panel or a keyboard. A controller 36 controls operations of respective units of the terminal apparatus 12.

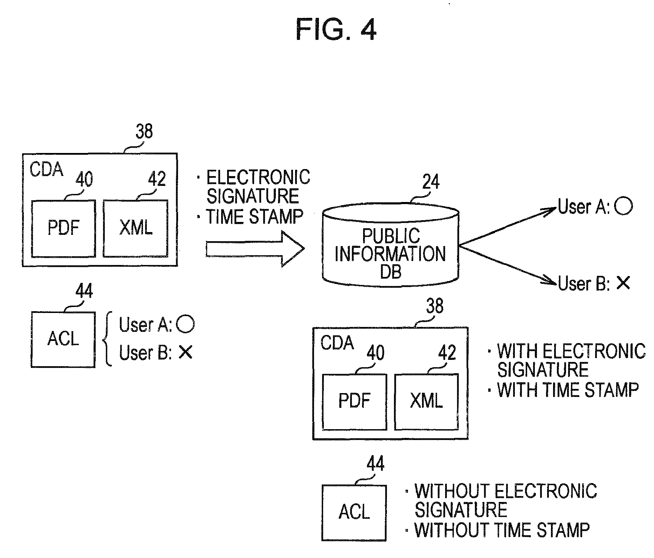

Processing in the medical information management system is described below with reference to Fig. 4. Fig. 4 is an illustration for describing the processing.

For example, a case conforming to the CDA standard is described. In this case, CDA information 38 is created and stored in the medical information DB 18. The CDA information 38 includes PDF data 40 serving as document data, and XML data 42 including information provided by the CDA standard. The PFD data 40 is, for example, data, such as an electronic chart and a letter of referral. The XML data 42 includes, for example, target information (patient information), report creation institution information, inspection information, interview information, etc. The PDF data 40 is treated as an attached document of the XML data 42. When the PDF data 40 is registered as document data of a publication object, access control information (ACL) 44 is created by the access control information creating unit 20, and is associated with the CDA information 38. For example, the PDF data 40 of a publication object and a publication destination are designated by a user of a publication source. In the example shown in Fig. 4, a user A is designated as a publication destination. That is, an access to the PDF data 40 from the user A is permitted; however, an access to the PDF data 40 from a user B is not permitted. In this case, the access control information (ACL) 44 includes, as publication destination information, identification information for identifying the user A (for example, a user ID).

The electronic signature processor 22 applies an electronic signature and time stamp processing to the CDA information 38. Accordingly, the electronic signature and the time stamp processing are applied to the PDF data 40 and the XML data 42. Of course, the electronic signature may be applied while the time stamp processing is not applied. Alternatively, the time stamp processing may be applied while the electronic signature is not applied. In contrast, the electronic signature processor 22 does not apply the electronic signature or the time stamp processing to the access control information (ACL) 44.

In the public information DB 24, the CDA information 38 (the PDF data 40 and the XML data 42) to which the electronic signature and the time stamp processing are applied, and the access control information (ACL) 44 to which the electronic signature or the time stamp processing is not applied are associated and stored.

When a user requests an access to the PDF data 40 by using the terminal apparatus 14, the access controller 28 controls the access by using the access control information (ACL) 44. For example, identification information for identifying the user is transmitted from the terminal apparatus 14 to the medical information management apparatus 10. If the identification information is included in the access control information (ACL) 44, the access to the PDF data 40 is permitted. If the identification information is not included in the access control information (ACL) 44, the access to the PDF data 40 is not permitted. For example, the access from the user A is permitted, however, the access from the user B is not permitted.

As described above, in this exemplary embodiment, the electronic signature and the time stamp processing are applied to the document data serving as the medical information. Accordingly, forging and tampering of the document data are prevented. In the case conforming to the CDA standard, the electronic signature and the time stamp processing are applied to the CDA information 38 (the PDF data 40 and the XML data 42). Accordingly, forging and tampering of the CDA information 38 are prevented. In contrast, the electronic signature or the time stamp processing is not applied to the access control information (ACL) 44. Accordingly, even when the access control to the document data (for example, the PDF data 40) is changed and hence the access control information (ACL) 44 is changed, it is not required to apply the electronic signature or the time stamp processing due to the change to prevent forging and tampering of the document data. When the electronic signature is applied, for example, it is required to execute calculation of hash information and making an access to a certificate authority, processing efficiency is decreased accordingly. In this exemplary embodiment, it is not required to apply the electronic signature or the time stamp processing due to the change in the access control information (ACL) 44, the decrease in processing efficiency caused by the electronic signature and the time stamp processing is prevented.

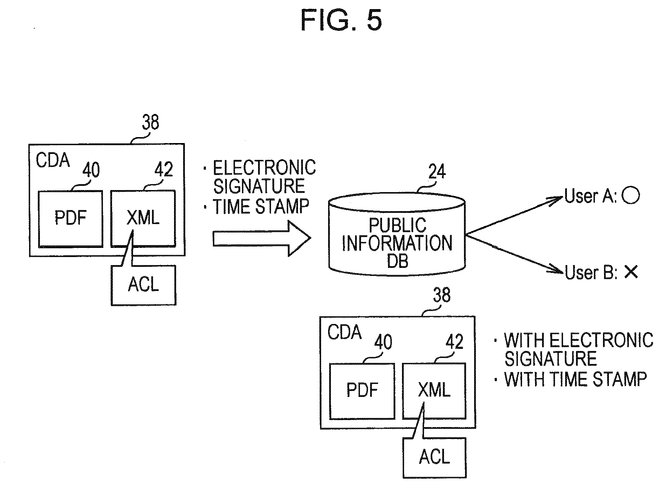

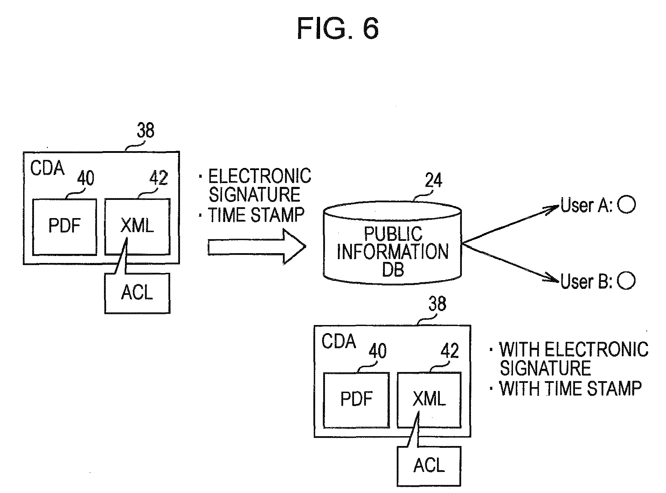

A comparative example is described below with reference to Figs. 5 and 6. Figs. 5 and 6 are illustrations for describing processing according to the comparative example.

In this comparative example, the case conforming to the CDA standard is also described. As shown in Fig. 5, in the comparative example, the access control information (ACL) is written in the XML data 42, and the electronic signature and the time stamp processing are applied to the PDF data 40 and the XML data 42. The PDF data 40 and the XML data 42 are stored in the public information DB 24. In an example shown in Fig. 5, the PDF data 40 is publicized to the user A, but is not publicized to the user B.

Fig. 6 illustrates processing when the PDF data 40 is also publicized to the user B according to the comparative example. When the PDF data 40 is publicized to the users A and B, the access control information (ACL) is changed. Since the access control information (ACL) is written in the XML data 42, the access control information (ACL) included in the XML data 42 is changed. To prevent forging and tampering of the XML data 42, the electronic signature and the time stamp processing are applied to the XML data 42 after the change. In this way, in the comparative example, every time when the access control is changed, it is required to apply the electronic signature and the time stamp processing due to the change.

In this exemplary embodiment, since the access control information (ACL) 44 is created independently from the CDA information 38 (the PDF data 40 and the XML data 42), every time when the access control is changed, it is not required to apply the electronic signature or the time stamp processing due to the change. Therefore, the decrease in processing efficiency is prevented as compared with the comparative example. For example, even when the PDF data 40 is publicized to the user B, it is only required to change the access control information (ACL) 44 independent from the CDA information 38, and the electronic signature or the time stamp processing is not required.

For example, when the publication destination is frequently changed, it is required to frequently change the access control information (ACL). If the access control information (ACL) is written in the XML data 42 itself, it is required to apply the electronic signature to the XML data 42 due to the change in the access control information (ACL). Even if the PDF data 40 itself serving as the document data is not changed, since the access control information (ACL) is written in the XML data 42, it is required to apply the electronic signature to the XML data 42 due to the change in the access control information (ACL). Accordingly, processing efficiency is decreased.

In contrast, in this exemplary embodiment, the access control information (ACL) 44 is created as data separated from the XML data 42 without being written in the XML data 42, and hence the access control information (ACL) 44 is not the object of the electronic signature and the time stamp processing. Therefore, even if the publication destination is changed and the access control information (ACL) 44 is changed, it is not required to apply the electronic signature or the time stamp processing to the CDA information 38. Even if the publication destination is frequently changed, it is not required to apply the electronic signature or the time stamp processing every time when the publication destination is changed to prevent forging and tampering of the CDA information 38. Accordingly, the decrease in processing efficiency is prevented as compared with a case in which the access control information (ACL) is written in the CDA information 38.

Since the access control information (ACL) is information expected to be frequently changed, by excluding the access control information (ACL) from the object to which the electronic signature and the time stamp processing are applied, the decrease in processing efficiency due to the electronic signature and the time stamp processing, which may frequently occur, is prevented.

If the access control information (ACL) 44 stored in the public information DB 24 is updated, the access control information (ACL) 44 may be stored in the public information DB 24 without deleting the previous access control information (ACL) 44 before the update. In this case, the access to the document data is controlled in accordance with the updated access control information (ACL) 44.

The access control information (ACL) may be written in the XML data 42, and also the access control information (ACL) 44 may be created independently from the CDA information 38 and associated with the CDA information 38. The electronic signature or the time stamp processing is not applied to the access control information (ACL) 44. In this case, the access control information (ACL) 44 independent from the CDA information 38 is used with a higher priority, and the access to the document data is controlled in accordance with the access control information (ACL) 44.

### Modification

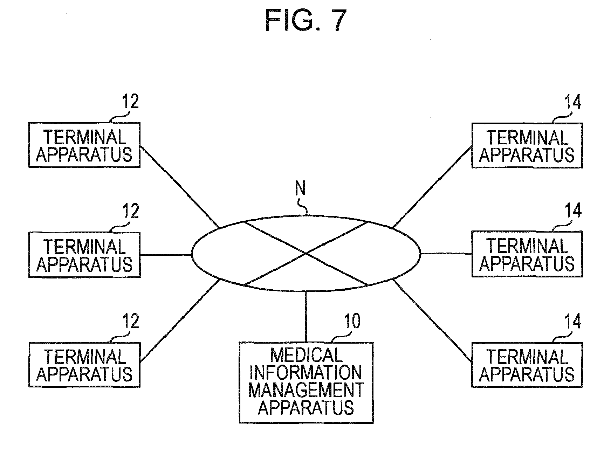

A modification is described below. Fig. 7 illustrates a medical information management system according to a modification. In the modification, plural terminal apparatuses 12 (for example, three terminal apparatuses 12) and plural terminal apparatuses 14 (for example, three terminal apparatuses 14) are connected to a communication path N. A medical information management apparatus 10 according to the modification has the same configuration as the configuration of the medical information management apparatus 10 according to the above-described exemplary embodiment. The modification is described below in detail.

In the modification, the access control information creating unit 20 creates access control information different depending on a public source group of document data of a publication object. A single piece of access control information may be created for a single piece of document data, and plural pieces of access control information may be created for a single piece of document data. For example, if access control is set for certain document data by a single publication source group, a single piece of access control information is created for the document data. In contrast, if access control is set for certain document data by plural publication source groups, plural pieces of access control information are created for the document data. In this case, access control information is created every publication source group, and consequently, access control information is created by the same number of pieces as the number of publication source groups.

The access control information includes, for example, publication source information relating to a publication source group, document management information about document data of a publication object, patient information associated with the document data, and publication destination information relating to a publication destination. The publication source group and the publication destination each may be, for example, a medical institution, a department in the medical institution, or an area of a prefecture or a municipality.

The publication destination information includes, for example, publication destination identification information for identifying a publication source group (for example, a publication source name or a publication source ID) and publication setting user identification information for identifying a publication setting user (for example, a publication source user name or a publication source user ID). If the unit of the publication source group is a medical institution, such as a hospital, a medical office, or a clinic, the publication source information includes, as the publication source identification information, medical institution identification information for identifying the medical institution (for example, a medical institution name or a medical institution ID). If the unit of the publication source group is a department, the publication source information includes, as the publication source identification information, medical institution identification information for identifying a medical institution to which the department belongs to (for example, a medical institution name or a medical institution ID) and department identification information for identifying the department (for example, a department name or a department ID). If the unit of the publication source group is an area, the publication source information includes, as the publication source identification information, area identification information for identifying the area (for example, an area name or an area ID). The publication source information may be transmitted from the terminal apparatus 12 that is used by the publication source group to the medical information management apparatus 10, for example, at publication processing, or may be previously stored in the medical information DB 18 of the medical information management apparatus 10.

The publication destination information is the same as the publication destination information according to the above-described exemplary embodiment.

For example, if a user such as a doctor designates document data of a publication object and a publication destination of the document data by using the terminal apparatus 12 in a publication source group, the access control information creating unit 20 creates access control information for controlling an access to the document data in accordance with the designation.

As described above, the access control information includes the patient information and the publication source information. The access control information creating unit 20 permits a change in the access control information by the group indicated by the publication source information included in the access control information, but inhibits a change in the access control information by a group other than the group indicated by the publication source information. For example, if the publication source information included in the access control information includes department identification information indicative of a department A, a change in the access control information by a doctor belonging to the department A is permitted, but a change in the access control information by a doctor belonging to a department other than the department A is inhibited. For example, if a user such as a doctor inputs patient information and publication source information by using the terminal apparatus 12 and hence makes a request for an access to access control information, the access control information creating unit 20 permits an access to access control information including the input patient information and publication source information, and permits a change in the access control information. In contrast, the access control information creating unit 20 inhibits an access to access control information not including the input patient information or publication source information, and inhibits a change in the access control information. In this way, the access control information is managed individually on a group basis.

Even in the modification, similarly to the above-described exemplary embodiment, the electronic signature processor 22 applies an electronic signature to document data. In the case conforming to the CDA standard, the electronic signature processor 22 applies an electronic signature to PDF data serving as document data, and applies the electronic signature to XML data. The electronic signature processor 22 does not apply the electronic signature to access control information (ACL). Also, the electronic signature processor 22 may apply time stamp processing to document data. In the case conforming to the CDA standard, the electronic signature processor 22 applies time stamp processing to PDF data serving as document data, and applies the time stamp processing to XML data. The electronic signature processor 22 does not apply the time stamp processing to the access control information (ACL).

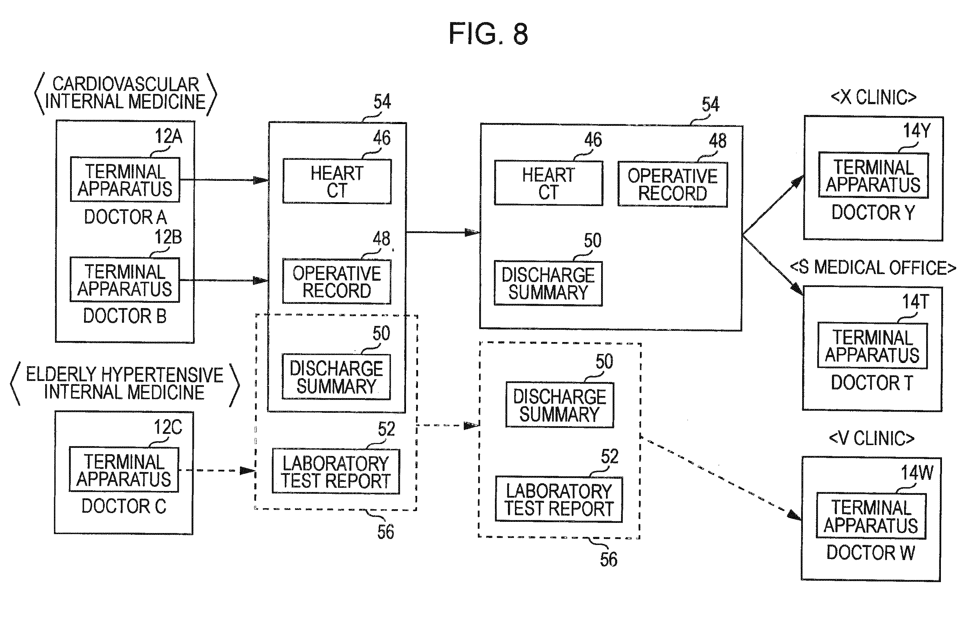

An overview of processing in the medical information management system according to the modification is described below with reference to Fig. 8. Fig. 8 is an illustration for describing the overview of the processing.

Terminal apparatuses 12A, 12B, and 12C are examples of the terminal apparatus 12, and are terminal apparatuses that are used in, for example, a medical institution of a publication source (for example, a university hospital). Cardiovascular internal medicine and elderly hypertensive internal medicine are examples of departments in the university hospital, and correspond to examples of publication source groups. The terminal apparatuses 12A and 12B are terminal apparatuses that are used in the cardiovascular internal medicine in the university hospital, and the terminal apparatus 12C is a terminal apparatus that is used in the elderly hypertensive internal medicine in the same university hospital.

Terminal apparatuses 14Y, 14T, and 14W are examples of the terminal apparatus 14, and are terminal apparatuses that are used in medical institutions of publication destinations. To be more specific, the terminal apparatus 14Y is a terminal apparatus that is used in an X clinic as a medical institution, the terminal apparatus 14T is a terminal apparatus that is used in an S medical office as a medical institution, and the terminal apparatus 14W is a terminal apparatus that is used in a V clinic as a medical institution. Publication destination information (medical institution information) about the X clinic, S medical office, and V clinic are previously stored in the medical information DB 18 of the medical information management apparatus 10.

For example, it is assumed that diagnosis and treatment are executed on, for example, a patient P, and a heart CT image 46, operative record information 48, discharge summary information 50, and a laboratory test report 52 are created as document data (medical information). The heart CT image 46, the operative record information 48, the discharge summary information 50, and the laboratory test report 52 each are associated with patient information on the patient P and stored in the medical information DB 18. The document data are data shared in, for example, the university hospital, and data permitted to be accessed from the terminal apparatuses 12A, 12B, and 12C. In the case conforming to the CDA standard, the heart CT image 46, the operative record information 48, the discharge summary information 50, and the laboratory test report 52 each are associated with XML data and stored in the medical information DB 18. An electronic signature and time stamp processing are applied to the heart CT image 46, the operative record information 48, the discharge summary information 50, and the laboratory test report 52. Also, the electronic signature and the time stamp processing are applied to the XML data associated with each document data.

The heart CT image 46, the operative record information 48, and the discharge summary information 50 are document data belonging to a publication document group 54. The discharge summary information 50 and the laboratory test report 52 are document data belonging to a publication document group 56.

The document data belonging to the publication document group 54 is data designated as publication document data by doctors A and B belonging to the cardiovascular internal medicine. The document data belonging to the publication document group 56 is data designated as publication document data by a doctor C belonging to the elderly hypertensive internal medicine. In the example shown in Fig. 8, the discharge summary information 50 belongs to both the publication document group 54 and the publication document group 56. That is, the discharge summary information 50 is designated as document data of a publication object by both the doctors of the cardiovascular internal medicine and the elderly hypertensive internal medicine.

By the doctors A and B belonging to the cardiovascular internal medicine, the X clinic and the S medical office are designated as medical institutions of publication destinations. Therefore, the document data belonging to the publication document group 54 is data permitted to be accessed from the X clinic (the terminal apparatus 14Y) and the S medical office (the terminal apparatus 14T).

Also, by the doctor C belonging to the elderly hypertensive internal medicine, the V clinic is designated as a medical institution of a publication destination. Therefore, the document data belonging to the publication document group 56 is data permitted to be accessed from the V clinic (the terminal apparatus 14W).

As described above, the department of the publication source, the document data of the publication object, and the medical institution of the publication destination are associated with each other, and the document data of the publication object and the medical institution of the publication destination are individually set every department of the publication source.

The medical information management apparatus 10 according to the modification is described below in further detail.

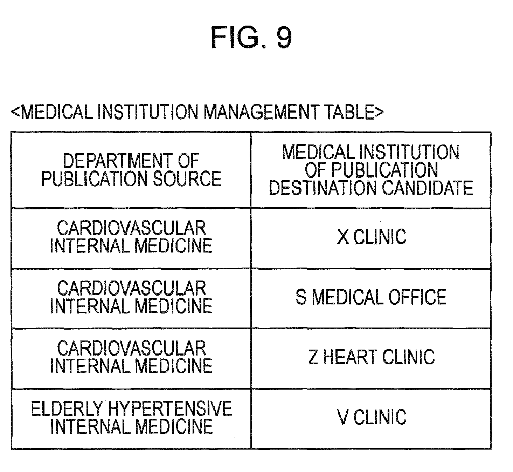

Examples of a medical institution registered in the medical information management system are described with reference to Fig. 9. Fig. 9 illustrates an example of a medical institution management table. The medical institution management table is an example of medical institution information. The data of the medical institution management table is previously created and stored in the medical information DB 18. A department of a publication source is an example of a publication source group of document data. As examples of the department of the publication source, cardiovascular internal medicine and elderly hypertensive internal medicine are registered. The cardiovascular internal medicine and the elderly hypertensive internal medicine are, for example, departments in the same university hospital. A medical institution of a publication destination candidate is a medical institution of a publication destination candidate of document data. As examples of the medical institution of the publication destination candidate, an X clinic, an S medical office, a Z heart clinic, and a V clinic are previously registered. In the example shown in Fig. 9, the cardiovascular internal medicine is associated with the X clinic, the S medical office, and the Z heart clinic, and the elderly hypertensive internal medicine is associated with the V clinic. That is, the X clinic, the S medical office, and the Z heart clinic are registered as medical institutions of publication destination candidates of document data to be publicized by a user such as a doctor belonging to the cardiovascular internal medicine, and the V clinic is registered as a medical institution of a publication destination candidate of document data to be publicized by a user such as a doctor belonging to the elderly hypertensive internal medicine. To select a medical institution of a publication destination, the user belonging to the cardiovascular internal medicine selects a medical institution of a publication destination from the X clinic, the S medical office, and the Z heart clinic. The user belonging to the elderly hypertensive internal medicine selects the V clinic as a medical institution of a publication destination.

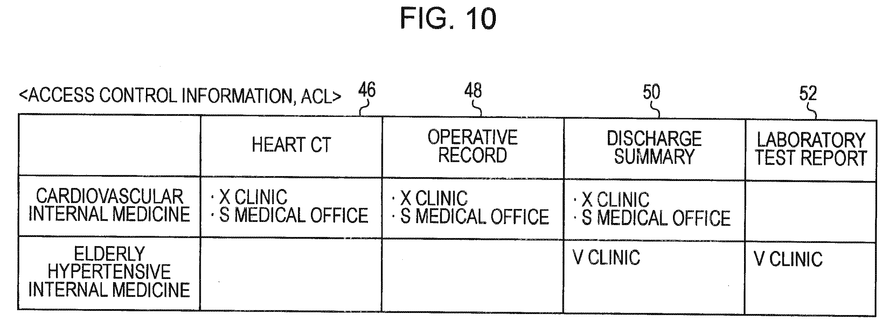

Access control information is described below in detail with reference to Fig. 10. Fig. 10 illustrates an example of access control information (ACL). The access control information is created in response to an instruction of a user of a publication source, and stored in the public information DB 24. Access control information being different depending on a publication source group is created. If the unit of the publication source group is a department, access control information being different depending on a department is created. In the example shown in Fig. 10, the heart CT image 46, the operative record information 48, and the discharge summary information 50 are designated as document data of a publication object of a patient P, and the X clinic and the S medical office are designated as medical institutions of publication destinations by a user belonging to the cardiovascular internal medicine of a publication source. Accordingly, in access control information created in response to the instruction of the user belonging to the cardiovascular internal medicine, patient information on a patient P, publication source information on the cardiovascular internal medicine, document management information about the document data of the publication object (the heart CT image 46, the operative record information 48, and the discharge summary information 50), and publication destination information on medical institutions of the publication destinations (the X clinic and the S medical office) are associated with each other. Also, the discharge summary information 50 and the laboratory test report 52 are designated as document data of a publication object of the patient P by a user belonging to the elderly hypertensive internal medicine of a publication source, and the V clinic is designated as a medical institution of a publication destination. Accordingly, in the access control information created in response to the instruction of the user belonging to the elderly hypertensive internal medicine, patient information on the patient P, publication source information on the elderly hypertensive internal medicine, document management information about the document data of the publication object (the discharge summary information 50 and the laboratory test report 52), and publication destination information on the medical institution of the publication destination (the V clinic) are associated with each other. In this way, the different pieces of access control information are created for the cardiovascular internal medicine and the elderly hypertensive internal medicine. The access controller 28 controls an access from a medial institution of a publication destination to document data by referencing the access control information.

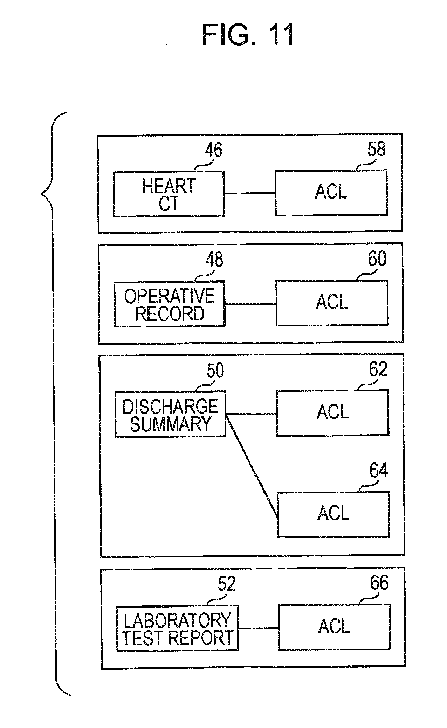

Fig. 11 illustrates an example of correspondence between document data and access control information (ACL). Access control information is created every document data of a publication object, and the document data and the access control information are associated with each other and stored in the public information DB 24.

For example, the heart CT image 46 and ACL 58 for controlling an access to the heart CT image 46 are associated with each other and stored in the public information DB 24. The ACL 58 includes patient information on a patient P, publication source information on the cardiovascular internal medicine of a publication source, document management information on the heart CT image 46, and publication destination information on medical institutions of publication destinations (the X clinic and the S medical office). In the case conforming to the CDA standard, an electronic signature and time stamp processing are applied to the heart CT image 46 and XML data. The electronic signature or the time stamp processing is not applied to the ACL 58.

Similarly, the operative record information 48 and ACL 60 for controlling an access to the operative record information 48 are associated with each other and stored in the public information DB 24. The ACL 60 includes patient information on a patient P, publication source information on the cardiovascular internal medicine of a publication source, document management information on the operative record information 48, and publication destination information on medical institutions of publication destinations (the X clinic and the S medical office). In the case conforming to the CDA standard, an electronic signature and time stamp processing are applied to the operative record information 48 and XML data. The electronic signature or the time stamp processing is not applied to the ACL 60.

Similarly, the discharge summary information 50, and ACL 62 and ACL 64 for controlling an access to the discharge summary information 50 are associated with each other and stored in the public information DB 24. As described above with reference to Figs. 8 and 10, the discharge summary information 50 is designated as the document data of the publication object by both the doctors of the cardiovascular internal medicine and the elderly hypertensive internal medicine. Therefore, the ACL 62 for the cardiovascular internal medicine and the ACL 64 for the elderly hypertensive internal medicine are created. The ACL 62 includes patient information on a patient P, publication source information on the cardiovascular internal medicine of a publication source, document management information on the discharge summary information 50, and publication destination information on medical institutions of publication destinations (the X clinic and the S medical office). Also, the ACL 64 includes patient information on a patient P, publication source information on the elderly hypertensive internal medicine of a publication source, document management information on the discharge summary information 50, and publication destination information on a medical institution of a publication destination (the V clinic). In the case conforming to the CDA standard, an electronic signature and time stamp processing are applied to the discharge summary information 50 and XML data. The electronic signature or the time stamp processing is not applied to the ACL 62 or the ACL 64.

Alternatively, the ACL 62 and the ACL 64 may be included in single ACL. In this case, in the single ACL, an access from a user belonging to the cardiovascular internal medicine is permitted for a portion having written therein the ACL 62, and a change in the portion is permitted. In contrast, in the single ACL, an access from a user belonging to the elderly hypertensive internal medicine is permitted for a portion having written therein the ACL 64, and a change in the portion is permitted.

Similarly, the laboratory test report 52 and ACL 66 for controlling an access to the laboratory test report 52 are associated with each other and stored in the public information DB 24. The ACL 66 includes patient information on a patient P, publication source information on the elderly hypertensive internal medicine of a publication source, document management information on the laboratory test report 52, and publication destination information on a medical institution of a publication destination (the V clinic). In the case conforming to the CDA standard, an electronic signature and time stamp processing are applied to the laboratory test report 52 and XML data. The electronic signature or the time stamp processing is not applied to the ACL 66.

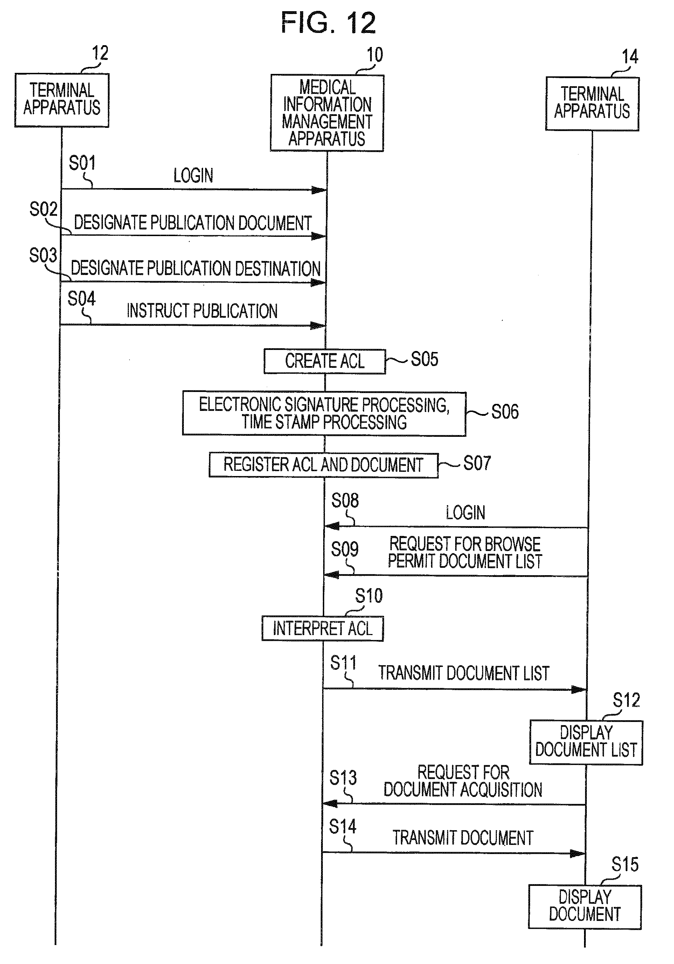

Processing in the medical information management system according to the modification is described below in detail with reference to Fig. 12. Fig. 12 is a sequence diagram showing the processing.

First, a user of a publication source logs in the medical information management apparatus 10 by using the terminal apparatus 12 (S01). For example, it is assumed that the doctor A of the cardiovascular internal medicine logs in the medical information management apparatus 10 by using a user ID and a password for the cardiovascular internal medicine. Authentication at login is executed by, for example, the controller 26 of the medical information management apparatus 10. If a user ID and a password previously registered in the medical information management apparatus 10 match the user ID and the password input from the terminal apparatus 12, login is permitted. In case of mismatch, login is not permitted.

Then, the user of the publication source (the doctor A of the cardiovascular internal medicine) designates document data of a publication object by using the terminal apparatus 12 (S02). For example, a list of document data stored in the medical information DB 18 is displayed on the UI unit 34 of the terminal apparatus 12. Document data of a publication object is designated by the user (the doctor A of the cardiovascular internal medicine) from the list.

Also, the user of the publication source (the doctor A of the cardiovascular internal medicine) designates a publication destination by using the terminal apparatus 12 (S03). For example, a medical institution of a publication destination is designated from a medical institution group registered in the medical information management system. If the user of the publication source is the doctor A of the cardiovascular internal medicine, a medical institution of a publication destination is designated from a medical institution group of publication destination candidates corresponding to the cardiovascular internal medicine. In the example shown in Fig. 9, the X clinic, the S medical office, and the Z heart clinic are registered as medical institutions of publication destination candidates corresponding to the cardiovascular internal medicine. For example, a list of the medical institutions of the publication destination candidates is displayed on the UI unit 34 of the terminal apparatus 12. A medical institution of a publication destination is designated by the user (the doctor A of the cardiovascular internal medicine) from the list.

Then, the user of the publication source (the doctor A of the cardiovascular internal medicine) instructs publication of the document data by using the terminal apparatus 12 (S04).

In the medical information management apparatus 10, the access control information creating unit 20 creates access control information (ACL) (S05). For example, as shown in Fig. 10, it is assumed that the doctor A of the cardiovascular internal medicine designates the heart CT image 46, the operative record information 48, and the discharge summary information 50 as document data of a publication object, and designates the X clinic and the S medical office as medical institutions of publication destinations. In this case, the access control information creating unit 20 creates access control information (ACL) for the heart CT image 46, access control information (ACL) for the operative record information 48, and access control information (ACL) for the discharge summary information 50. For example, as shown in Fig. 11, the ACL 58 is created for the heart CT image 46, the ACL 60 is created for the operative record information 48, and the ACL 62 is created for the discharge summary information 50.

Then, the electronic signature processor 22 applies an electronic signature and time stamp processing to the heart CT image 46, the operative record information 48, and the discharge summary information 50 (S06). The electronic signature or the time stamp processing is not applied to the ACL 58, 60, or 62. In the case conforming to the CDA standard, the electronic signature processor 22 applies the electronic signature and the time stamp processing to XML data associated with each document data.

Document data of a publication object is associated with access control information for the document data and stored in the public information DB 24 (S07). In the case conforming to the CDA standard, the XML data is also associated with the document data and stored in the public information DB 24.

Even when document data is publicized by the doctor C of the elderly hypertensive internal medicine, processing similar to the above-described processing is executed. Accordingly, access control information for the elderly hypertensive internal medicine is created. For example, as shown in Fig. 10, it is assumed that the doctor C of the elderly hypertensive internal medicine designates the discharge summary information 50 and the laboratory test report 52 as document data of publication objects, and designates the V clinic as a medical institution of a publication destination. In this case, the access control information creating unit 20 creates access control information (ACL) for the discharge summary information 50 and access control information (ACL) for the laboratory test report 52. For example, as shown in Fig. 11, the ACL 64 is created for the discharge summary information 50, and the ACL 66 is created for the laboratory test report 52. Even in this case, an electronic signature and time stamp processing are applied to the discharge summary information 50 and the laboratory test report 52, but the electronic signature or the time stamp processing is not applied to the ACL 64 or the ACL 66. In the case conforming to the CDA standard, XML data for the discharge summary information 50 and XML data for the laboratory test report 52 are created, and the electronic signature and the time stamp processing are applied to each XML data.

When a user of a publication destination makes an access to document data, the user of the publication destination logs in the medical information management apparatus 10 by using the terminal apparatus 14 (S08). For example, it is assumed that the doctor Y of the X clinic logs in the medical information management apparatus 10 by using a user ID and a password. Authentication at login is executed by, for example, the controller 26 of the medical information management apparatus 10. If a user ID and a password previously registered in the medical information management apparatus 10 match the user ID and the password input from the terminal apparatus 14, login is permitted. In case of mismatch, login is not permitted.

Then, the user of the publication destination (the doctor Y of the X clinic) makes a request for a list of document data permitted to be browsed by the user, by using the terminal apparatus 14 (S09). At this time, publication destination information is transmitted from the terminal apparatus 14 to the medical information management apparatus 10. The publication destination information includes medical institution identification information for identifying the X clinic (for example, the name or ID of the X clinic).

In the medical information management apparatus 10, the access controller 28 interprets access control information (ACL) stored in the public information DB 24 (S10). Accordingly, the access controller 28 specifies document data permitted to be browsed by the user of the publication destination (the doctor Y of the X clinic). To be specific, the access controller 28 references access control information including medical institution identification information transmitted from the terminal apparatus 14 as publication destination information, and specifies document data associated with the medical institution identification information, as document data permitted to be browsed by the user of the publication destination. Then, the access controller 28 transmits data of a list of document data permitted to be browsed by the user of the publication destination, to the terminal apparatus 14 (S11). The list of the document data permitted to be browsed is displayed on the UI unit 34 of the terminal apparatus 14 (S12).

For example, as shown in Fig. 10, the heart CT image 46, the operative record information 48, and the discharge summary information 50 are publicized to the X clinic. Accordingly, an access to the heart CT image 46, the operative record information 48, and the discharge summary information 50 is permitted. In this case, the access controller 28 transmits the data of the list of the document data permitted to be publicized to the X clinic, to the terminal apparatus 14. Accordingly, the list of the heart CT image 46, the operative record information 48, and the discharge summary information 50 is displayed on the UI unit 34 of the terminal apparatus 14 of the X clinic.

Then, the user of the publication destination (the doctor Y of the X clinic) designates document data being an acquisition object from the list of the document data displayed on the UI unit 34 and makes a request for acquisition of the document data by using the terminal apparatus 14 (S13). Accordingly, information for identifying the document data designated by the user is transmitted from the terminal apparatus 14 to the medical information management apparatus 10.

In the medical information management apparatus 10, the access controller 28 acquires the document data being the acquisition object, which is designated by the user, from the public information DB 24, and transmits the document data to the terminal apparatus 14 (S14). In the terminal apparatus 14, the document data is displayed on the UI unit 34 (S 15).

For example, when the heart CT image 46 is designated by the doctor Y of the X clinic, data (document data) of the heart CT image 46 is transmitted from the medical information management apparatus 10 to the terminal apparatus 14, and the heart CT image 46 is displayed on the UI unit 34 of the terminal apparatus 14.

Even when the doctor T of the S medical office or the doctor W of the V clinic is a user of a publication destination, processing similar to the above-described processing is executed. For example, when a request is made for acquisition of document data by the doctor T of the S medical office, a list of the heart CT image 46, the operative record information 48, and the discharge summary information 50 is displayed on the UI unit 34 of the terminal apparatus 14, as a list of document data permitted to be browsed. When a request is made for acquisition of document data by the doctor W of the V clinic, a list of the discharge summary information 50 and the laboratory test report 52 is displayed on the UI unit 34 of the terminal apparatus 14, as a list of document data permitted to be browsed.

As described above, in the modification, different access control information is generated on a publication source group basis, and an access to document data is controlled based on the access control information. The access control information is individually managed on a group basis, and an access to access control information set by one group from the other group is inhibited. Accordingly, the access control information set by the one group is not changed by the other group. Therefore, on the basis of a publication source group of document data, access control to the document data is provided.

For example, access control information set by a doctor belonging to the doctor's department is not changed by a doctor belonging to another department. Accordingly, on the basis of a department of a publication source of document data, access control to the document data is provided. For example, when respective departments are independent in the same hospital, even if the same medical information management system is used, independence of each department is ensured for access control to document data.

Also, if access control information is created every group, the access control information is changed every group. Hence, the frequency of change in access control information is expected to be increased. Even in the modification, an electronic signature or time stamp processing is not applied to access control information (ACL). Therefore, even if the access control information of each group is frequently changed, the electronic signature or the time stamp processing is not required to be applied every time when the access control information of each group is changed. As compared with a case in which access control information is written in CDA information, the decrease in processing efficiency is prevented.

In the above-described example, a department is a publication source group, and access control information is created every individual department. However, even when the publication source group is a medical institution or an area, independence of each group is ensured for access control to document data similarly to the case of the department. For example, if plural medical institutions are registered in the medical information management system as publication source groups, access control information is created every medical institution, and access control information set by one medical institution is not changed by the other medical institution. Accordingly, even when the same medical information management system is used, independence of each medical institution is ensured for access control to document data.

If the publication source group is a medical institution or an area, it may be expected that the medical information management system according to the modification is applied to local medical care. In this case, by using the medical information management system according to the modification, access control to medical information in the local medical care is provided.

With the exemplary embodiment and the modification, by updating access control information without updating document data itself, access control to document data is changed. For example, when plural publication source groups set access control to the same document data in accordance with the convenience of each group, only access control information may be created or updated in accordance with the convenience of each group without updating the document data itself.

In the above-described exemplary embodiment and modification, when publication processing is applied to document data stored in the medical information DB 18, a template named default publication document type may be used. The default publication document type is a type of document data of a publication object, and the template indicates the type. For example, when a user belonging to a publication source group logs in the medical information management apparatus 10 and instructs application of publication processing, the access control information creating unit 20 specifies the type of document data of a publication object by referencing the template and treats the document data corresponding to the default publication document type as document data of a publication object. Document management information on document data includes a document type code indicative of a document type. By referencing the document type code, the type of the document data is specified. Access control information about the publication source group includes document management information on the document data corresponding to the default publication document type and publication destination information about a publication destination designated by a user belonging to the publication source group. Accordingly, an access from the publication destination to the document data corresponding to the default publication document type is permitted. For example, if the default publication document type is an electronic chart, the electronic chart is treated as a publication object. By using the default publication document type, as compared with a case without the use of the default publication document type, work of the user relating to the publication processing is simplified.

As another example, when the publication processing is applied to document data which will be registered in the medical information DB 18 in future, a template named automatic publication document type may be used. The automatic publication document type is a type of document data of a publication object, and the template indicates the type. When document data is newly stored in the medical information DB 18, the access control information creating unit 20 specifies the type of the document data of the publication object by referencing the template. If the type of the newly registered document data corresponds to the pubic document type, the access control information creating unit 20 treats the document data as a publication object. In this case, access control information includes document management information on the document data. By using the automatic publication document type, as compared with a case without the use of the automatic publication document type, work of a user relating to the publication processing is simplified.

Also, a publication period of document data may be set. The publication period is a period in which an access to document data from a publication destination is permitted. The access controller 28 permits an access to the document data from the publication destination in the publication period, and inhibits an access to the document data from the publication destination outside the publication period. Information indicative of the publication period is included in access control information. The access controller 28 controls an access depending on the publication period by referencing the information. Accordingly, an access to the document data is automatically inhibited when the publication period has elapsed. The user of the publication source does not have to stop the publication.

When at least one of a publication source group, document data of a publication object, and a state of a publication destination is changed, access control information relating to the change is updated, or new access control information is created. For example, if at least one of states including a publication flag of document data, a publication period, a publication destination (medical institution, medical office, area, etc.), a publication destination permitted for publication by a publication source group, a publication destination facility master, an automatic publication document type, an automatic publication period, a default publication document type of the publication source group, a document type permitted for publication by the publication source group, a defined document type, a department, a patient or a user, document data, a version of the document data, a publication tag of the document data, a document type, is changed, access control information relating to the change in the state is updated or new access control information is created. The other access control information not relating to the above-described change in the state is not influenced, or updated by the change in the state. That is, individual control information is independent from the other access control information. Even when a state relating to certain access control information is changed, the other access control information is not influenced, or updated by the change in the state.

When publication of document data is stopped, document data of a publication stop object may be deleted from the public information DB 24, and access control information associated with the document data may be left in the public information DB 24. Accordingly, leakage of the document data of the publication stop object may be prevented, and information relating to access control may be provided to the publication destination.

The medical information management apparatus 10 is realized by, for example, cooperation of a hardware resource and software. To be specific, the medical information management apparatus 10 includes a processor such as a central processing unit (CPU) (not illustrated). The processor reads out a program stored in a memory device (not shown) and executes the program. Hence, the functions of the respective units of the medical information management apparatus 10 are realized. The program is stored in the memory device through a storage medium, such as a compact disc (CD) or a digital versatile disc (DVD), or a communication path such as a network. Alternatively, the respective units of the medical information management apparatus 10 may be realized by, for example, a hardware resource, such as a processor or an electronic circuit. A device such as a memory may be used for the realization. For another example, the respective units of the medical information management apparatus 10 may be realized by, for example, a digital signal processor (DSP) or a field programmable gate array (FPGA).

The foregoing description of the exemplary embodiment of the present invention has been provided for the purposes of illustration and description. It is not intended to be exhaustive or to limit the invention to the precise forms disclosed. Obviously, many modifications and variations will be apparent to practitioners skilled in the art. The embodiment was chosen and described in order to best explain the principles of the invention and its practical applications, thereby enabling others skilled in the art to understand the invention for various embodiments and with the various modifications as are suited to the particular use contemplated. It is intended that the scope of the invention be defined by the following claims and their equivalents.

## Claims

1. An information processing apparatus comprising:
a memory that stores document data;
a creating unit that creates access control information for controlling an access to the document data;
a processor that applies an electronic signature to the document data, but does not apply the electronic signature to the access control information; and
a controller that controls the access to the document data by using the access control information.

2. The information processing apparatus according to Claim 1, wherein the document data is data relating to medical care.

3. The information processing apparatus according to Claim 1 or 2, wherein the access control information is information indicative of a publication destination of the document data.

4. The information processing apparatus according to any one of Claims 1 to 3, wherein the creating unit creates the access control information different depending on a group of a publication source of the document data.

5. The information processing apparatus according to any one of Claims 1 to 4, wherein the processor further applies time stamp processing to the document data.

6. A program causing a computer including a memory that stores document data to function as a configuration, the configuration comprising:
a creating unit that creates access control information for controlling an access to the document data;
a processor that applies an electronic signature to the document data, but does not apply the electronic signature to the access control information; and
a controller that controls the access to the document data by using the access control information.

7. An information processing method comprising:
storing document data;
creating access control information for controlling an access to the document data;
applying an electronic signature to the document data, but not applying the electronic signature to the access control information; and
controlling the access to the document data by using the access control information.
